# EUROPÄISCHE PATENTSCHRIFT

(11) **EP 4 054 745 B1**
(45) Veröffentlichungstag und Bekanntmachung des Hinweises auf die Patenterteilung: **16.04.2025**
(21) Anmeldenummer: 20803113.8
(22) Anmeldetag: 03.11.2020
(51) Int. Cl.: B01D 65/10, A61M 1/16, B01D 61/24, B01D 63/02

(54) **VERFAHREN UND ANLAGE ZUM PRÜFEN DER INTEGRITÄT EINES KAPILLARDIALYSATORS**
METHOD AND INSTALLATION FOR TESTING THE INTEGRITY OF A CAPILLARY DIALYSER
PROCÉDÉ ET INSTALLATION PERMETTANT DE TESTER L'INTÉGRITÉ D'UN DIALYSEUR CAPILLAIRE

(30) Priorität: 04.11.2019 DE 102019129664
(43) Veröffentlichungstag der Anmeldung: 14.09.2022
(73) Patentinhaber: Fresenius Medical Care Deutschland GmbH, 61352 Bad Homburg (DE)
(72) Erfinder: PITSCH, Stefan, 66287 Quierschied (DE); WILHELM, Manuel, 66386 St. Ingbert (DE)
(74) Vertreter: Stolmár & Partner Patentanwälte PartG mbB
(86) Internationale Anmeldenummer: PCT/EP2020/080843
(87) Internationale Veröffentlichungsnummer: WO 2021/089565

(56) Entgegenhaltungen:
- DE-A1- 4 215 585
- DE-C2- 4 215 585
- JP-A- 2001 099 775
- US-B1- 6 324 898

## Beschreibung

Die Erfindung betrifft ein Verfahren zum Prüfen der Integrität eines Flüssigkeitskapillarfilters, insbesondere eines Kapillardialysators, welcher aus einer Vielzahl von durch eine Membran umschlossenen Kapillaren aufgebaut ist, mit den Schritten: Durchströmen der Innen- oder Außenseite der Kapillaren mit einer Flüssigkeit, Beaufschlagen der jeweils korrespondierenden Außen- oder Innenseite der Kapillaren mit einem Gas, wobei das Gas einen höheren Druck aufweist als die Flüssigkeit, und Bestimmen einer Menge des Gases, welche durch Undichtigkeiten der Membran in die Flüssigkeit eindringt.

Weiterhin betrifft die Erfindung eine Anlage zum Prüfen der Integrität eines Flüssigkeitskapillarfilters, insbesondere eines Kapillardialysators, welcher aus einer Vielzahl von durch eine Membran umschlossenen Kapillaren aufgebaut ist, umfassend: ein Spülsystem welches eingerichtet ist, eine Flüssigkeit durch das Innere oder Äußere der Kapillaren eines zur prüfenden Kapillardialysators zu leiten, ein Gasdrucksystem welches eingerichtet ist, das jeweils korrespondierende Äußere oder Innere der Kapillaren des zu prüfenden Flüssigkeitskapillarfilters, insbesondere des Kapillardialysators, mit einem Gas zu beaufschlagen, wobei der Druck des Gases höher ist als der Druck der Flüssigkeit, und eine Messeinrichtung zur Bestimmung einer während eines vorgegebenen oder vorgebbaren Referenzzeitraums durch Undichtigkeiten der Membran in die Flüssigkeit eindringenden Gasmenge.

Aus dem Stand der Technik sind Verfahren und Anlagen zur Durchführung von Integritätstests bekannt, beispielsweise aus US 6 324 898 B1, DE 42 15 585 A1 und JP 2001 099775 A.

Kapillardialysatoren werden in der Medizin eingesetzt, um bei Patienten mit eingeschränkter Nierenfunktion Schadstoffe aus dem Blut zu filtern. Dazu wird das mit Schadstoffen belastete Blut über eine semipermeable Membran in Kontakt mit einer Dialyseflüssigkeit gebracht. Die Dialyseflüssigkeit weist eine verschwindend geringe Konzentration der zu entfernenden Schadstoffe wie z.B. Harnstoff auf, so dass die Schadstoffe aufgrund eines Konzentrationsgefälles durch die Membran in die Dialyseflüssigkeit diffundieren oder aufgrund konvektiver Effekte in die Dialyseflüssigkeit gelangen. Gleichzeitig ist die Membran für andere Blutbestandteile wie Blutplasma oder partikuläre Bestandteile zumindest teilweise undurchlässig. Dieser Vorgang wird als Dialyse bezeichnet.

Andere Flüssigkeitskapillarfilter werden häufig ebenfalls in der Medizintechnik eingesetzt, beispielsweise um Rohwasser für die Dialyse aufzubereiten. Solche Filter können auch sonst in der Wasseraufbereitung verwendet werden.

Insbesondere für eine wirksame Blutreinigung ist eine große Oberfläche der Membran erforderlich. Dazu weisen bekannte Kapillardialysatoren eine Vielzahl von Kapillaren auf, beispielsweise ca. 10.000. Die Wände der Kapillaren werden durch eine semipermeable Membran gebildet. Die Kapillaren sind als lockeres Bündel in einem rohrartigen Volumen verlegt.

Während der Dialyse wird das Blut des Patienten durch die Kapillaren geleitet, während durch das rohrartige Volumen die Dialyseflüssigkeit strömt. Die Strömungsrichtungen von Blut und Dialyseflüssigkeit sind dabei vorzugsweise entgegengesetzt, der Dialysator arbeitet also im Gegenstromprinzip.

Kapillardialysatoren werden in der Regel als sterile Einmalprodukte bereitgestellt, und werden dazu nach der Herstellung einem Sterilisationsverfahren unterzogen. Hier findet oftmals eine Dampfsterilisation Anwendung. Auch andere Flüssigkeitskapillarfilter werden häufig mit Dampf sterilisiert.

Da die Kapillaren eine sehr geringe Wandstärke aufweisen, kann es vorkommen, dass bei der Herstellung eines Flüssigkeitskapillarfilters oder Kapillardialysators Undichtigkeiten einzelner Kapillaren auftreten. Außerdem können Undichtigkeiten durch verschiedene andere Schadenstypen entstehen. Durch solche Undichtigkeiten kann während der Dialyse einerseits Blut aus den Kapillaren in die Dialyseflüssigkeit gelangen, und andererseits Dialyseflüssigkeit in das Blut eindringen. Beides ist unerwünscht, wobei besonders das Eindringen von Dialyseflüssigkeit in das Blut des Patienten in größeren Mengen physiologisch problematisch sein kann.

Um Flüssigkeitskapillarfilter, insbesondere Kapillardialysatoren, mit entsprechenden Undichtigkeiten zu erkennen, werden diese einer Prüfung unterzogen. Da Undichtigkeiten auch während der Sterilisierung entstehen können, wird die Prüfung in der Regel unmittelbar nach der Sterilisierung durchgeführt.

In einem bekannten Prüfverfahren für Kapillardialysatoren wird eine Prüfflüssigkeit, zumeist Wasser, durch die Kapillaren geleitet, während gleichzeitig ein Gas in das die Kapillaren umgebende Volumen eingeleitet wird. Das Gas steht dabei unter einem höheren Druck als die Flüssigkeit, so dass es durch etwaige Undichtigkeiten in die Kapillaren eindringen kann.

Dabei bildet das Gas in der Flüssigkeit kleine Bläschen, welche durch den Flüssigkeitsstrom aus den Kapillaren gespült werden. Um die Bläschen nachzuweisen, wird der Flüssigkeitsstrom anschließend durch ein Schauglas geleitet, welches von einer Kamera beobachtet wird.

Mit dieser Methode ist jedoch eine quantitative Bestimmung der eingedrungenen Gasmenge schwierig, da jede einzelne Blase während des Vorbeiströmens bewertet werden muss und die Form der Blasen nicht vollständig erfasst werden kann. Zudem muss das Schauglas über einen längeren Zeitraum überwacht werden, um eine ausreichende Sensitivität zu erreichen. Wenn zudem die Prüfung auf einem getakteten Förderer über mehrere Rastpositionen erfolgt, muss die Beobachtung unterbrochen werden und es können einzelne Blasen leicht übersehen werden.

Es besteht daher eine Aufgabe der Erfindung darin, ein verbessertes Verfahren und eine verbesserte Anlage zum Prüfen der Integrität eines Flüssigkeitskapillarfilters, insbesondere eines Kapillardialysators, bereitzustellen.

Diese Aufgabe wird gemäß eines ersten Aspekts der Erfindung gelöst durch ein Verfahren zum Prüfen der Integrität eines Flüssigkeitskapillarfilters, insbesondere eines Kapillardialysators, welcher aus einer Vielzahl von durch eine Membran umschlossenen Kapillaren aufgebaut ist, mit den Schritten: Durchströmen der Innenseite der Kapillaren mit einer Flüssigkeit und Beaufschlagen der Außenseite der Kapillaren mit einem Gas, oder Durchströmen der Außenseite der Kapillaren mit einer Flüssigkeit und Beaufschlagen der Innenseite der Kapillaren mit einem Gas, wobei das Gas jeweils einen höheren Druck aufweist als die Flüssigkeit, und Bestimmen einer Menge des Gases, welche durch Undichtigkeiten der Membran in die Flüssigkeit eindringt, welches dadurch weitergebildet ist, dass die Flüssigkeit nach dem Durchströmen der Kapillaren durch eine Blasenfalle geleitet wird, und dass ein sich während eines vorgegebenen oder vorgebbaren Referenzzeitraums in der Blasenfalle ansammelndes Gasvolumen bestimmt wird.

Es werden hierbei die einzelnen in die Kapillaren eintretenden oder aus den Kapillaren austretenden Gasbläschen in der Blasenfalle gesammelt, so dass sie ein weitgehend, bevorzugt einheitliches Gasvolumen bilden. Dieses Gasvolumen kann einfach ermittelt werden, so dass auch ohne eine Langzeitbeobachtung eine genaue quantitative Bestimmung der eindringenden Gasmenge möglich ist.

Es ist somit möglich, die Außenseite der Kapillaren oder die Innenseite mit dem Gas zu beaufschlagen, wobei die jeweils komplementäre Seite dann mit der Flüssigkeit beaufschlagt wird.

Bevorzugt ist der Flüssigkeitskapillarfilter ein Kapillardialysator. Die Membranen dieser Dialysatoren weisen eine besonders geringe Wandstärke von 10 bis 50 µm, bevorzugt 15 bis 35 µm, auf, so dass eine besondere Leckanfälligkeit besteht. Die Anforderungen an die Leckfreiheit sind zudem bei Dialysatoren aufgrund der geforderten Patientensicherheit besonders hoch. Gleichzeitig hat sich gezeigt, dass die austretenden Gasbläschen besonders feingliedrig und damit optisch als Einzelbläschen schwer detektierbar sind. Für Kapillardialysatoren ist das erfindungsgemäße Verfahren somit besonders vorteilhaft anwendbar.

Insbesondere bei Kapillardialysatoren enthaltend Membranen mit der selektiven Oberfläche im Lumen der Membran ist es bevorzugt, die Außenseite mit Gas und die Innenseite mit Flüssigkeit zu beaufschlagen. Dadurch ist es möglich, eine besonders schonende Behandlung des Lumens der Membran während des Lecktests zu gewährleisten.

In einer vorteilhaften Weiterbildung eines Verfahrens gemäß der Erfindung kann zu Beginn des Referenzzeitraums ein Anfangswert des Gasvolumens bestimmt werden, am Ende des Referenzzeitraums ein Endwert des Gasvolumens bestimmt werden, und aus der Differenz zwischen dem Anfangswert und dem Endwert kann die in dem Referenzzeitraum in die Kapillaren eingedrungene Gasmenge bestimmt werden.

Als Flüssigkeit kann vorzugsweise Wasser verwendet werden. Durch die Verwendung von Wasser als Testflüssigkeit kann vermieden werden, dass ggf. Lösemittelgemische als Abfall aus dem Verfahren anfallen. Zudem können Restmengen von Wasser ohne gesundheitliche Beeinträchtigungen des Patienten nach dem durchgeführten Test in einem Filter, insbesondere in einem Dialysator, verbleiben.

In einer besonders vorteilhaften Umsetzung eines Verfahrens nach der Erfindung kann ein die Blasenfalle und den Flüssigkeitskapillarfilter, insbesondere den Kapillardialysator, umfassendes Leitungssystem vor dem Beginn der Prüfung mit Wasserdampf beaufschlagt werden.

Eine Beaufschlagung des Leitungssystems mit Wasserdampf kann erfolgen, um den Flüssigkeitskapillarfilter, insbesondere den Kapillardialysator, sowie mit diesem temporär verbundene Leitungsabschnitte zu sterilisieren. Dabei füllt der Wasserdampf die Blasenfalle aus, wobei ggf. von einem vorangegangenen Prüfvorgang darin enthaltenes Gas verdrängt wird. Sobald der Wasserdampf erkaltet und kondensiert, kollabiert das Dampfvolumen in der Blasenfalle, so dass diese annähernd vollständig mit Wasser befüllt werden kann, ohne dass hierzu ein Ventil benötigt wird.

Die Bestimmung des in der Blasenfalle angesammelten Gasvolumens kann in einer bevorzugten Umsetzung eines Verfahrens nach der Erfindung optisch erfolgen. Dazu kann die Blasenfalle einen transparenten Abschnitt aufweisen, und zur Bestimmung des in der Blasenfalle angesammelten Gasvolumens kann die Lage einer Flüssigkeitsoberfläche in dem transparenten Abschnitt ausgewertet werden. Optische verfahren haben sich als besonders robust, d.h. wenig störanfällig, und einfach erwiesen.

Die Lage der Flüssigkeitsoberfläche in dem transparenten Abschnitt lässt sich nach optischer Detektion mittels Bildverarbeitung unkompliziert ermitteln.

In einer vorteilhaften Ausführungsform eines Verfahrens gemäß der Erfindung kann zu Beginn des Referenzzeitraums wenigstens ein erstes Kamerabild des transparenten Abschnitts aufgenommen und die anfängliche Lage der Flüssigkeitsoberfläche mittels eines Bildauswerteverfahrens bestimmt werden, am Ende des Referenzzeitraums wenigstens ein zweites Kamerabild des transparenten Abschnitts aufgenommen und die abschließende Lage der Flüssigkeitsoberfläche mittels eines Bildauswerteverfahrens bestimmt werden, und aus der anfänglichen Lage der Flüssigkeitsoberfläche und der abschließenden Lage der Flüssigkeitsoberfläche kann das angesammelte Gasvolumen bestimmt werden.

Das erste und das zweite Kamerabild können vorzugsweise in einem Durchlichtverfahren aufgenommen werden. Durchlichtverfahren lassen sich mit geringem apparativem Aufwand bei geringem Platzbedarf in einer kompakten Fertigungsanlage realisieren.

Dabei kann die Beleuchtung nach einer bevorzugten Ausführungsform mittels einer fluoreszierenden Folie erfolgen, welche mit Anregungslicht einer ersten Wellenlänge beleuchtet wird, und welche in Reaktion auf die Beleuchtung mit Anregungslicht Fluoreszenzlicht einer zweiten Wellenlänge aussendet, wobei die zweite Wellenlänge größer ist als die erste Wellenlänge, und die Kamerabilder können durch ein für Licht der ersten Wellenlänge intransparentes Filter aufgenommen werden.

Bei einer entsprechenden Beleuchtung sind einerseits störende Reflexionen an den Oberflächen des transparenten Abschnitts auf ein Minimum reduziert, während gleichzeitig Kamera und Lichtquelle auf der gleichen Seite des transparenten Abschnitts angeordnet sein können. Hierdurch wird der Platzaufwand für die Messeinrichtung weiter reduziert.

Ein die Blasenfalle und den Kapillardialysator umfassendes Strömungssystem kann dabei an einem getaktet betriebenen Förderer angeordnet sein, welcher während des Referenzzeitraums um wenigstens eine Förderposition weiterbewegt wird.

Die Aufgabe wird gemäß eines weiteren Aspekts der Erfindung gelöst durch eine Anlage zum Prüfen der Integrität eines Flüssigkeitskapillarfilters, insbesondere eines Kapillardialysators, welcher aus einer Vielzahl von durch eine Membran umschlossenen Kapillaren aufgebaut ist, umfassend ein Spülsystem, welches eingerichtet ist, eine Flüssigkeit durch das Innere der Kapillaren eines zu prüfenden Flüssigkeitskapillarfilters zu leiten und ein Gasdrucksystem welches eingerichtet ist, dass Äußere der Kapillaren des zu prüfenden Flüssigkeitskapillarfilters mit einem Gas zu beaufschlagen, oder ein Spülsystem, welches eingerichtet ist, eine Flüssigkeit durch das Äußere der Kapillaren eines zu prüfenden Filters zu leiten und ein Gasdrucksystem welches eingerichtet ist, das Innere der Kapillaren des zu prüfenden Filters mit einem Gas zu beaufschlagen, wobei der Druck des Gases jeweils höher ist als der Druck der Flüssigkeit, und eine Messeinrichtung zur Bestimmung einer während eines vorgegebenen oder vorgebbaren Referenzzeitraums durch Undichtigkeiten der Membran in die Flüssigkeit eindringenden Gasmenge, welche dadurch weitergebildet ist, dass das Spülsystem eine stromabwärts des Flüssigkeitskapillarfilters angeordnete Blasenfalle umfasst, und dass das Messsystem eingerichtet ist, ein sich während eines vorgegebenen oder vorgebbaren Referenzzeitraums in der Blasenfalle ansammelndes Gasvolumen zu bestimmen.

Es ist somit möglich, die Außenseite der Kapillaren oder die Innenseite mit dem Gas zu beaufschlagen, wobei die jeweils komplementäre Seite dann mit der Flüssigkeit beaufschlagt wird.

Bevorzugt ist der Flüssigkeitskapillarfilter ein Kapillardialysator. Die Membranen dieser Dialysatoren weisen eine besonders geringe Wandstärke von 10 bis 50 µm, bevorzugt 15 bis 35 µm, auf, so dass eine besondere Leckanfälligkeit besteht. Die Anforderungen an die Leckfreiheit sind zudem bei Dialysatoren besonders hoch.

Insbesondere bei Kapillardialysatoren enthaltend Membranen mit der selektiven Oberfläche im Lumen der Membran ist es bevorzugt, die Außenseite mit Gas und die Innenseite mit Flüssigkeit zu beaufschlagen, Dadurch ist es möglich, eine besonders schonende Behandlung des Lumens der Membran während des Lecktestes zu gewährleisten.

In einer vorteilhaften Ausführung einer Anlage gemäß der Erfindung kann die Messeinrichtung eingerichtet sein, zu Beginn des Referenzzeitraums einen Anfangswert des Gasvolumens zu bestimmen, am Ende des Referenzzeitraums ein Endwert des Gasvolumens zu bestimmen, und aus der Differenz zwischen dem Anfangswert und dem Endwert die in dem Referenzzeitraum in die Kapillaren eingedrungene Gasmenge zu bestimmen.

In einer besonderen Ausführungsform weist die Blasenfalle einen Fangabschnitt im Strömungskanal der Testflüssigkeit auf, so dass sich die Strömungsgeschwindigkeit der Testflüssigkeit zumindest kurzfristig, beispielsweise für 100 ms bis 10 sec., verringert. Dadurch können sich Bläschen besonders effektiv aus der Testflüssigkeit lösen und der Volumendetektion zugeführt werden. Bevorzugt werden die Bläschen durch eine obere Wandung des Fangabschnittes zu einem Schauglas geleitet. Bevorzugt ist die obere Wandung kegelförmig ausgebildet.

Die Messeinrichtung kann gemäß einer weiteren Ausgestaltung einer erfindungsgemäßen Anlage eingerichtet sein, das sich in der Blasenfalle ansammelnde Gasvolumen optisch zu bestimmen. Dazu kann die Blasenfalle einen transparenten Abschnitt aufweisen, und die Messeinrichtung kann eingerichtet sein, zur Bestimmung des in der Blasenfalle angesammelten Gasvolumens die Lage einer Flüssigkeitsoberfläche in dem transparenten Abschnitt auszuwerten.

In einer bevorzugten Ausführung einer Anlage gemäß der Erfindung kann die Messeinrichtung wenigstens eine Kamera und ein Bildauswertungssystem umfassen. Dabei kann die Messeinrichtung wenigstens eine fluoreszierende Folie und wenigstens eine Quelle für Anregungslicht umfassen.

Alternativ zur optischen Detektion kann auch eine alternative Füllstandsdetektion vorgesehen sein, beispielsweise mit Hilfe eines Ultraschall- Füllstandssensors.

In einer weiteren Ausführungsform der Erfindung kann die Anlage Bestandteil eines getaktet angetriebenen Fördersystems sein, welches mehrere Förderelemente umfasst, wobei jedes Förderelement zur Aufnahme mindestens eines Flüssigkeitskapillarfilters, insbesondere eines Kapillardialysators, vorgesehen ist, und wobei jedes Förderelement für jeden aufzunehmenden Flüssigkeitskapillarfilter eine Blasenfalle umfasst, welche gemeinsam mit dem Flüssigkeitskapillarfilter entlang des Fördersystems bewegbar ist.

Die Messeinrichtung kann eine erste Kamera umfassen, welche angrenzend an einen Förderweg des Fördersystems stationär angeordnet ist. Die Messeinrichtung kann weiterhin eine zweite Kamera umfassen, welche angrenzend an den Förderweg des Fördersystems stationär angeordnet ist. Die erste und/oder die zweite Kamera können vorzugsweise angrenzend an eine Rastposition des Fördersystems angeordnet sein.

Das Fördersystem kann ein Rundförderer sein.

Die Erfindung wird nachfolgend anhand einiger beispielhafter Figuren näher erläutert, wobei die in den Figuren dargestellten Ausführungsformen lediglich zum besseren Verständnis der Erfindung dienen sollen, ohne diese einzuschränken.

Es zeigen:
Fig. 1: einen Kapillardialysator als Beispiel für einen Flüssigkeitskapillarfilter in einer vereinfachten Schnittdarstellung,
Fig. 2: eine Kapillare mit einer Undichtigkeit,
Fig. 3: eine Messeinrichtung mit Blasenfalle,
Fig. 4: einen Rundförderer.

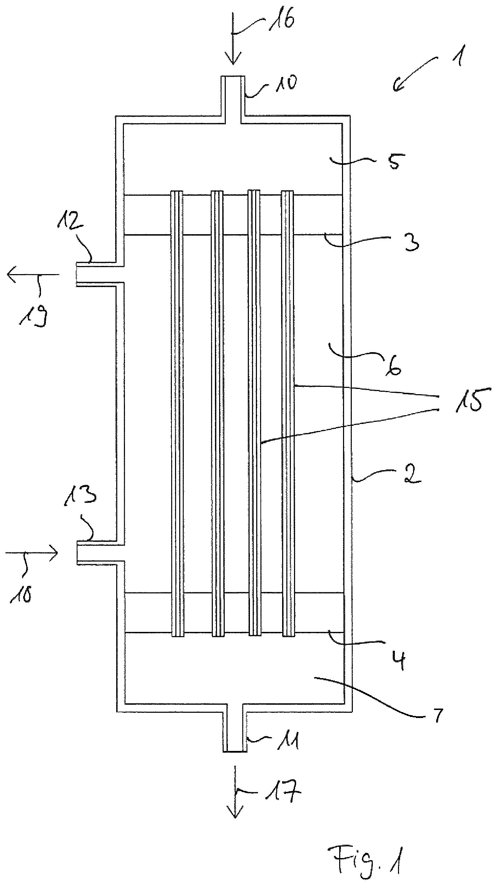

In Figur 1 ist ein Kapillardialysator 1 in einer vereinfachten Schnittdarstellung abgebildet. Der Kapillardialysator 1 besteht aus einem in etwa zylindrischen Gehäuse 2, welches durch Dichteinsätze 3, 4 in drei Kammern 5, 6, 7 unterteilt ist. In Abweichung vom Ausführungsbeispiel kann statt eines Kapillardialysators auch ein Flüssigkeitskapillarfilter vorgesehen sein.

Die endseitigen Kammern 5, 7 weisen jeweils einen Anschlussstutzen 10, 11 auf. Die mittlere Kammer 6 weist zwei Anschlussstutzen 12, 13 auf, die jeweils nahe der Dichteinsätze 3, 4 angeordnet sind. Bei den Anschlussstutzen 10, 11, 12, 13 kann es sich beispielsweise um "Luer-Lock" Anschlüsse handeln.

Kapillaren 15 verlaufen von der Kammer 5 durch die Dichteinsätze 3, 4 und die mittlere Kammer 6 in die Kammer 7. Die Darstellung der Kapillaren 15 in der Figur 1 ist stark vereinfacht. Ein Kapillardialysator weist üblicherweise anstelle der in Figur 1 dargestellten vier Kapillaren bis zu 18000 Kapillaren auf, die in der Regel nicht vollständig gestreckt sind. Die Wandstärke der Kapillaren 15 kann ca. 10 - 50µm, insbesondere 15 bis 35 µm, betragen, der Innendurchmesser der Kapillaren 15 kann ca. 180 bis 300 µm betragen. Die Wandungen der Kapillaren 15 bestehen aus einer biokompatiblen semipermeablen Membran. Bevorzugt umfasst die Membran ein Polysulfonmaterial, welches mit Polyvinylpyrrolidon (PVP) modifiziert wird.

Wird der Filter für die Aufbereitung von Rohwasser verwendet, ist auch diese die Membran bevorzugt hydrophil, so dass eine gute Benetzbarkeit insbesondere mit Wasser gegeben ist.

Bei der Dialyse wird Blut entlang der Pfeile 16, 17 durch den Anschlussstutzen 10 in die Kammer 5 gepumpt und strömt dann durch den Innenraum der Kapillaren 15 in die Kammer 7. Von dort wird das Blut durch den Anschlussstutzen 11 wieder abgegeben und dem Patienten wieder zugeführt.

Parallel wird Dialyseflüssigkeit entlang der Pfeile 18, 19 durch den Anschlussstutzen 13 in die mittlere Kammer 6 geleitet, wo sie entgegen der Strömungsrichtung des Blutes entlang der Kapillaren 15 strömt, bevor sie durch den Anschlussstutzen 12 wieder abgegeben wird. Giftstoffe wie beispielsweise Harnstoff diffundieren dabei aus dem Blut durch die Membranhülle der Kapillaren 15 in die Dialyseflüssigkeit. Dadurch wird das Blut gereinigt.

Für die wirksame und sichere Funktion des Kapillardialysators 1 ist es erforderlich, dass die Strömungspfade für Blut und Dialyseflüssigkeit gegeneinander hinreichend abgedichtet sind. Undichtigkeiten können einen unkontrollierten Übertritt von Dialyseflüssigkeit in das Blut, sowie umgekehrt einen Verlust von Blut in die Dialyseflüssigkeit erlauben, was physiologisch unerwünscht ist.

Einige mögliche Quellen für Undichtigkeiten sind mechanische Fehler der Membranhüllen, die bei der Extrusion der Kapillaren 15 entstehen können, unzureichende Abdichtung zwischen den Dichteinsätzen 3, 4 und den Kapillaren 15 oder dem Gehäuse 2, oder gebrochene Kapillaren 15. Entsprechende Fehler können bei der Fertigung der Kapillarmembranen, des Kapillardialysators 1 oder bei einer anschließenden Sterilisierung auftreten.

Eine Sterilisierung des Kapillardialysators 1 ist erforderlich, da dieser während der Dialyse mit dem Blut des Patienten in Berührung kommt. Ein gängiges Verfahren zur Sterilisierung ist die Dampfsterilisierung, bei welcher heißer Wasserdampf durch den Kapillardialysator 1 geleitet wird. Der Wasserdampf durchströmt dabei sowohl den Strömungsweg des Blutes als auch den Strömungsweg der Dialyseflüssigkeit, so dass alle Oberflächen in dem Kapillardialysator erhitzt, beispielsweise auf über 124°C, und eventuell vorhandene Krankheitserreger abgetötet werden.

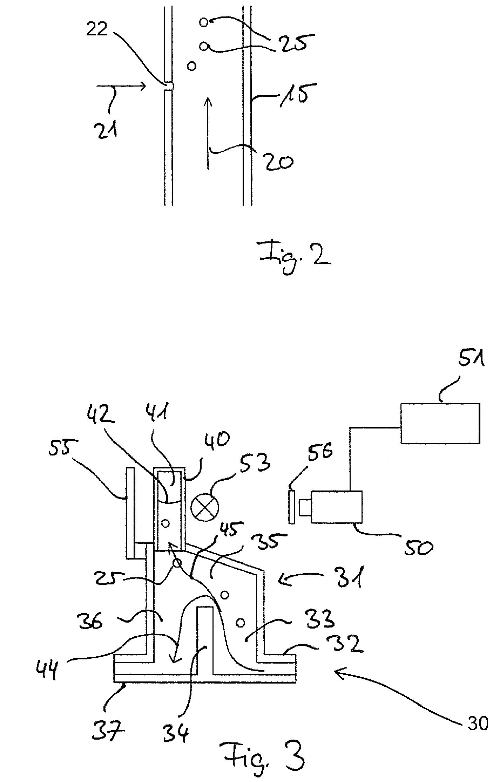

Um Kapillardialysatoren mit Undichtigkeiten von der Verwendung auszuschließen, werden die Kapillardialysatoren nach der Sterilisation einem Dichtigkeitstest unterzogen. Das Testprinzip ist in Figur 2 dargestellt.

Dazu wird eine Testflüssigkeit, zumeist Wasser, in Richtung des Pfeils 20 durch die Kapillaren 15 geleitet, während gleichzeitig ein Prüfgas, z.B. Luft, in die Kammer 6 eingeleitet wird. In Abweichung vom Ausführungsbeispiel kann auch vorgesehen sein, dass die Testflüssigkeit in die Kammer 6 eingeleitet wird, während das Prüfgas in die Kapillaren 15 geleitet wird. Dabei steht das Prüfgas jeweils unter einem höheren Druck als die Testflüssigkeit, was durch den Pfeil 21 angedeutet ist. Weist nun der Kapillardialysator 1 eine Undichtigkeit 22 auf, so kann Prüfgas durch diese Undichtigkeit 22 in die Testflüssigkeit eindringen und dort Bläschen 25 bilden. Diese Bläschen werden mit der Testflüssigkeit aus der Kapillare 15 gespült und können anschließend nachgewiesen werden.

Zum qualitativen und quantitativen Nachweis der Bläschen 25 wird die Testflüssigkeit nach Durchlaufen des Kapillardialysators 1 durch eine Blasenfalle 30 geleitet. Diese ist in Figur 3 dargestellt.

Die Blasenfalle 30 besteht aus einer Strömungskammer 31, in welche die Testflüssigkeit durch einen Zulauf 32 einströmt. An den Zulauf 32 angrenzend weist die Strömungskammer einen ansteigenden Abschnitt 33 auf, welcher durch einen Trennkörper 34 begrenzt ist. Oberhalb des Trennkörpers 34 geht der aufsteigende Abschnitt 33 in einen Fangabschnitt 35 über. An den Fangabschnitt 35 schließt sich ein abfallender Abschnitt 36 an, welcher in einen Ablauf 37 übergeht.

Der Fangabschnitt 35 ist so ausgelegt, dass sich die Strömungsgeschwindigkeit der Testflüssigkeit hier kurzzeitig verringert. Dabei lösen sich die Bläschen 25 aus dem Strom der Testflüssigkeit und werden durch eine kegelförmige obere Wandung des Fangabschnitts 35 zu einem Schauglas 40 geleitet.

In dem Schauglas 40 sammeln sich die Bläschen 25 zu einem zusammenhängenden Gasvolumen 41. Zwischen dem Gasvolumen 41 und der Testflüssigkeit bildet sich eine Grenzfläche 42 aus, welche zur optischen Messung des Gasvolumens 41 genutzt wird.

Die Strömungswege der Testflüssigkeit und der Bläschen 25 sind in der Figur durch Pfeile 44, 45 angedeutet.

Zur Messung des Gasvolumens 41 wird das Schauglas 40 durch eine Kamera 50 beobachtet, und auf einem bzw. auf mehreren nacheinander aufgenommenen Bildern der Kamera wird durch eine Bildverarbeitungseinheit 51 die Lage der Grenzfläche 42 bestimmt.

Um Reflexionen zu unterdrücken, wird die optische Messung des Gasvolumens 41 in einem Durchlichtverfahren durchgeführt. Da allerdings auf der in Figur 3 links dargestellten Rückseite des Schauglases 40 konstruktionsbedingt wenig Platz vorhanden ist, müssen trotzdem die Kamera 50 und eine Lichtquelle 53 auf der selben Seite des Schauglases 40 angeordnet sein. Die Lichtquelle 53 ist dabei so angeordnet, dass sie den Blick der Kamera 50 auf das Schauglas 40 nicht verdeckt. Dazu kann die Lichtquelle 40 vertikal oder horizontal leicht versetzt angeordnet sein, oder die Lichtquelle 40 kann als um die Blickrichtung Kamera 40 angeordnete Ringleuchte ausgeführt sein.

Um trotzdem mit Durchlicht arbeiten zu können, ist hinter dem Schauglas 40 eine fluoreszierende Folie 55 angeordnet. Die Lichtquelle 53 sendet Anregungslicht einer ersten Wellenlänge, beispielsweise im blauen Spektralbereich, aus, welches teilweise von dem Schauglas 40 in Richtung der Kamera 50 reflektiert wird. Ein Großteil des Anregungslichts fällt jedoch auf die fluoreszierende Folie 55 und wird dort in Fluoreszenzlicht einer zweiten Wellenlänge, beispielsweise im orangen Spektralbereich, umgewandelt. Das Fluoreszenzlicht wird in Richtung des Schauglases 40 und der Kamera 50 abgestrahlt.

Vor dem Objektiv der Kamera 50 ist ein Farbfilter 56 angeordnet, welcher für das kurzwellige Anregungslicht intransparent und für das Fluoreszenzlicht transparent ist. Demzufolge erreicht das an dem Schauglas 40 reflektierte Anregungslicht die Kamera 50 nicht, und das aufgenommene Bild ist frei von störenden Reflexionen. Auf einem entsprechend aufgenommenen Bild lässt sich die Grenzfläche 42 mit geringem Aufwand mittels hinlänglich bekannter Methoden der Bildauswertung auffinden und hinsichtlich Ihrer Lage bestimmen. Aus der Lage der Grenzfläche 42 kann wiederum einfach das Gasvolumen 41 ermittelt werden.

Eine entsprechende fluoreszierende Folie kann beispielsweise unter der Produktbezeichnung "CA-DWC30" von der Firma Keyence Corporation bezogen werden.

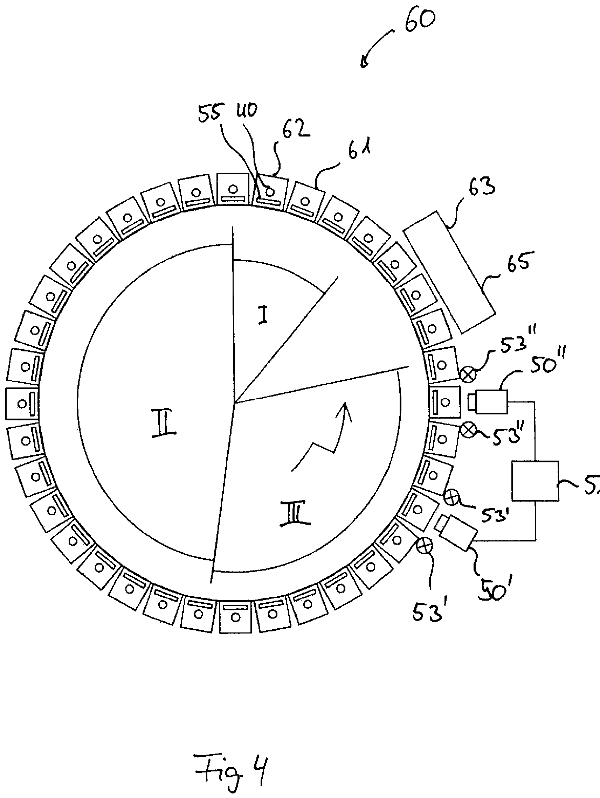

Die Sterilisierung und Prüfung von Kapillardialysatoren erfolgt auf einem getaktet arbeitenden Förderer, beispielsweise einem Rundförderer. Ein solcher Rundförderer 60 ist in Figur 4 schematisch dargestellt. Der Rundförderer 60 umfasst eine Vielzahl von individuellen Aufnahmen 61, 62 für jeweils einen oder mehrere Kapillardialysatoren 1. Jede der Aufnahmen 61, 62 umfasst für jeden aufzunehmenden Kapillardialysator 1 eine Blasenfalle 30, von denen hier jeweils die Schaugläser 40 und die fluoreszierenden Folien 55 sichtbar sind.

Die Kapillardialysatoren werden in einer Beladestation 63 in die Aufnahmen 61, 62 eingesetzt. Dabei bewegt sich der Rundförderer 60 in festgelegten Zeitabständen jeweils um eine Position weiter, so dass die nächste der Aufnahmen 61, 62 in der Beladestation 63 mit einem Kapillardialysator beladen werden kann.

Die Aufnahmen 61, 62 werden schrittweise entlang des Rundförderers 60 bewegt und durchlaufen dabei unter anderem einen Vorbereitungsabschnitt I, und einen Sterilisationsabschnitt II, in welchem heißer Dampf durch den Kapillardialysator und das Leitungssystem der Aufnahmen 61, 62 geleitet wird, um diese zu sterilisieren. Dabei dringt der Dampf bis in das Schauglas 40 vor, so dass auch dieses komplett mit Wasserdampf gefüllt und sterilisiert ist.

In einem Prüfabschnitt III wird der den Strömungsweg des Blutes bildende Abschnitt des Kapillardialysators mit sterilfiltriertem Wasser gespült. Dabei kollabiert der Dampf in dem Schauglas 40, so dass sich dieses vollständig mit Wasser füllt. Parallel wird der den Strömungsweg der Dialyseflüssigkeit bildende Abschnitt des Kapillardialysators mit sterilfiltrierter Luft gespült, um den Wasserdampf herauszudrücken. Optional kann auch hier vor dem Spülen mit sterilfiltrierter Luft ein Spülen mit sterilfiltriertem Wasser erfolgen.

Während des Durchlaufens des Spülabschnitts gelangen die Aufnahmen 61, 62 in eine erste Messposition, an welcher eine erste Kamera 50' und erste Lichtquellen 53' angeordnet sind. Die erste Kamera 50' nimmt ein oder mehrere Bilder des Schauglases 40 an der ersten Messposition auf und leitet diese an eine Bildauswerteeinheit 51 weiter. Durch die Bildauswertungseinheit 51 wird die Lage der Grenzfläche 42 in der ersten Messposition ermittelt und zusammen mit einer Kennung der individuellen Aufnahme 61, 62 gespeichert.

Gegen Ende des Spülabschnitts gelangen die Aufnahmen 61, 62 in eine zweite Messposition, an welcher eine zweite Kamera 50" und zweite Lichtquellen 53" vorgesehen sind. Die zweite Kamera 50" nimmt ein oder mehrere Bilder des Schauglases 40 an der zweiten Messposition auf und leitet diese ebenfalls an die Bildauswerteeinheit 51 weiter. Durch die Bildauswertungseinheit 51 wird die Lage der Grenzfläche 42 in der zweiten Messposition ermittelt.

Aus der Änderung der Lage der Grenzfläche 42 zwischen der ersten Messposition und der zweiten Messposition bestimmt die Bildauswerteeinheit 51 oder eine nachgeordnete Steuerung die während des Transports von der ersten Messposition zur zweiten Messposition in die Kapillaren 15 des Kapillardialysators 1 eingedrungene Luftmenge. Wenn die Taktrate des Rundförderers 60 bekannt und konstant ist, kann diese Luftmenge direkt als Kriterium für die Dichtigkeit des Kapillardialysators 1 verwendet werden. Ist die Taktrate nicht bekannt, so kann zusammen mit dem Messergebnis an der ersten Messposition ein erster Zeitstempel abgespeichert werden, und zusammen mit dem Messergebnis an der zweiten Messposition ein zweiter Zeitstempel abgespeichert werden. Aus der Differenz der beiden Zeitstempel kann dann die Durchlaufzeit des Kapillardialysators bestimmt und bei der Beurteilung berücksichtigt werden.

Liegt die eingedrungene Luftmenge bzw. die Leckrate über einem vorgegebenen Grenzwert, so muss der entsprechende Kapillardialysator als undicht bewertet und von der weiteren Verwendung ausgeschlossen werden.

Nach Durchlaufen des Spülabschnitts wird das Wasser aus dem Kapillardialysator gedrückt und er wird in einer Entladestation 65 von dem Rundförderer entnommen, so dass ein weiterer Kapillardialysator in die entsprechende Aufnahme 61,62 eingesetzt werden kann.

Die Beladestation 63 und die Entladestation 65 können, wie in Figur 4 dargestellt, zu einer Station zusammengefasst sein.

Die Darstellung des Rundläufers 60 in Figur 4 ist wiederum stark vereinfacht. Der Rundläufer 60 kann eine wesentlich höhere Anzahl an Aufnahmen 61, 62 aufweisen, um einen ausreichenden Durchsatz von Kapillardialysatoren 1 bereitzustellen. Zwischen dem Prüfabschnitt III und der Entladestation 65 kann ein Trocknungsabschnitt vorgesehen sein, der hier nicht dargestellt ist. Die relativen Längen der einzelnen Abschnitte sind in Figur 4 nicht zwangsläufig maßstabsgerecht abgebildet. Für die an sich bekannten Funktionen des Rundläufers benötigte Leitungen und Ventile sind zur besseren Übersichtlichkeit nicht dargestellt.

## Patentansprüche

1. Verfahren zum Prüfen der Integrität eines Flüssigkeitskapillarfilters (1), insbesondere eines Kapillardialysators , welcher aus einer Vielzahl von durch eine Membran umschlossenen Kapillaren (15) aufgebaut ist, mit den Schritten:
- Durchströmen der Innenseite der Kapillaren (15) mit einer Flüssigkeit und
- Beaufschlagen der Außenseite der Kapillaren (15) mit einem Gas, oder
- Durchströmen der Außenseite der Kapillaren (15) mit einer Flüssigkeit und
- Beaufschlagen der Innenseite der Kapillaren (15) mit einem Gas,
wobei das Gas jeweils einen höheren Druck aufweist als die Flüssigkeit, und
- Bestimmen einer Menge des Gases, welche durch Undichtigkeiten der Membran in die Flüssigkeit eindringt,
**dadurch gekennzeichnet, dass**
- die Flüssigkeit nach dem Durchströmen des Flüssigkeitskapillarfilters (1) durch eine Blasenfalle (30) geleitet wird, und dass
- ein sich während eines vorgegebenen oder vorgebbaren Referenzzeitraums in der Blasenfalle (30) ansammelndes Gasvolumen (41) bestimmt wird.

2. Verfahren nach Anspruch 1, **dadurch gekennzeichnet, dass**
- zu Beginn des Referenzzeitraums ein Anfangswert des Gasvolumens (41) bestimmt wird,
- am Ende des Referenzzeitraums ein Endwert des Gasvolumens (41) bestimmt wird, und
- dass aus der Differenz zwischen dem Anfangswert und dem Endwert die in dem Referenzzeitraum in die Flüssigkeit eingedrungene Gasmenge bestimmt wird.

3. Verfahren nach Anspruch 1 oder 2, **dadurch gekennzeichnet, dass** als Flüssigkeit Wasser verwendet wird.

4. Verfahren nach einem der Ansprüche 1 bis 3, **dadurch gekennzeichnet, dass** ein die Blasenfalle (30) und den Flüssigkeitskapillarfilter (1) umfassendes Leitungssystem vor dem Beginn der Prüfung mit Wasserdampf beaufschlagt wird.

5. Verfahren nach einem der Ansprüche 1 bis 4, **dadurch gekennzeichnet, dass** die Bestimmung des in der Blasenfalle (30) angesammelten Gasvolumens optisch erfolgt.

6. Verfahren nach Anspruch 5, **dadurch gekennzeichnet, dass** die Blasenfalle (30) einen transparenten Abschnitt (40) aufweist, und dass zur Bestimmung des in der Blasenfalle (30) angesammelten Gasvolumens (41) die Lage einer Flüssigkeitsoberfläche (42) in dem transparenten Abschnitt (40) ausgewertet wird.

7. Verfahren nach Anspruch 6, **dadurch gekennzeichnet, dass**
- zu Beginn des Referenzzeitraums wenigstens ein erstes Kamerabild des transparenten Abschnitts (40) aufgenommen und die anfängliche Lage der Flüssigkeitsoberfläche (42) mittels eines Bildauswerteverfahrens bestimmt wird,
- am Ende des Referenzzeitraums wenigstens ein zweites Kamerabild des transparenten Abschnitts (40) aufgenommen wird und die abschließende Lage der Flüssigkeitsoberfläche (42) mittels eines Bildauswerteverfahrens bestimmt wird, und dass
- aus der anfänglichen Lage der Flüssigkeitsoberfläche (42) und der abschließenden Lage der Flüssigkeitsoberfläche (42) das angesammelte Gasvolumen (41) bestimmt wird.

8. Verfahren nach Anspruch 7, **dadurch gekennzeichnet, dass** das erste und das zweite Kamerabild in einem Durchlichtverfahren aufgenommen werden.

9. Verfahren nach Anspruch 8, **dadurch gekennzeichnet, dass** die Beleuchtung mittels einer fluoreszierenden Folie (55) erfolgt, welche mit Anregungslicht einer ersten Wellenlänge beleuchtet wird, und welche in Reaktion auf die Beleuchtung mit Anregungslicht Fluoreszenzlicht einer zweiten Wellenlänge aussendet, wobei die zweite Wellenlänge größer ist als die erste Wellenlänge, und wobei die Kamerabilder durch ein für Licht der ersten Wellenlänge intransparentes Filter (56) aufgenommen werden.

10. Verfahren nach einem der Ansprüche 2 bis 9, **dadurch gekennzeichnet, dass** ein die Blasenfalle (30) und den Flüssigkeitskapillarfilter (1) umfassendes Strömungssystem an einem getaktet betriebenen Förderer (60) angeordnet sind, welcher während des Referenzzeitraums um wenigstens eine Förderposition weiterbewegt wird.

11. Anlage zum Prüfen der Integrität eines Flüssigkeitskapillarfilters (1), insbesondere eines Kapillardialysators, welcher aus einer Vielzahl von durch eine Membran umschlossenen Kapillaren (15) aufgebaut ist, umfassend:
- ein Spülsystem welches eingerichtet ist, eine Flüssigkeit durch das Innere der Kapillaren (15) eines zur prüfenden Flüssigkeitskapillarfilters (1) zu leiten und
- ein Gasdrucksystem welches eingerichtet ist, dass Äußere der Kapillaren (15) des zu prüfenden Flüssigkeitskapillarfilters (1) mit einem Gas zu beaufschlagen, oder
- ein Spülsystem welches eingerichtet ist, eine Flüssigkeit durch das Äußere der Kapillaren (15) eines zur prüfenden Flüssigkeitskapillarfilters (1) zu leiten und
- ein Gasdrucksystem welches eingerichtet ist, das Innere der Kapillaren (15) des zu prüfenden Flüssigkeitskapillarfilters (1) mit einem Gas zu beaufschlagen,
wobei der Druck des Gases jeweils höher ist als der Druck der Flüssigkeit, und
- eine Messeinrichtung zur Bestimmung einer während eines vorgegebenen oder vorgebbaren Referenzzeitraums durch Undichtigkeiten der Membran in die Flüssigkeit eindringenden Gasmenge (41),
**dadurch gekennzeichnet, dass**
- das Spülsystem eine stromabwärts des Flüssigkeitskapillarfilters (1) angeordnete Blasenfalle (30) umfasst, und
- dass die Messeinrichtung eingerichtet ist, ein sich während eines vorgegebenen oder vorgebbaren Referenzzeitraums in der Blasenfalle (30) ansammelndes Gasvolumen (41) zu bestimmen.

12. Anlage nach Anspruch 11, **dadurch gekennzeichnet, dass** die Messeinrichtung eingerichtet ist,
- zu Beginn des Referenzzeitraums einen Anfangswert des Gasvolumens (41) zu bestimmen,
- am Ende des Referenzzeitraums ein Endwert des Gasvolumens (41) zu bestimmen, und
- aus der Differenz zwischen dem Anfangswert und dem Endwert die in dem Referenzzeitraum in die Kapillaren (15) eingedrungene Gasmenge zu bestimmen.

13. Anlage nach Anspruch 11 oder 12, **dadurch gekennzeichnet, dass** die Messeinrichtung eingerichtet ist, das sich in der Blasenfalle (30) ansammelnde Gasvolumen (41) optisch zu bestimmen.

14. Anlage nach Anspruch 13, **dadurch gekennzeichnet, dass** die Blasenfalle (30) einen transparenten Abschnitt (40) aufweist, und dass die Messeinrichtung eingerichtet ist, zur Bestimmung der in der Blasenfalle (30) angesammelten Gasvolumens (41) die Lage einer Flüssigkeitsoberfläche (42) in dem transparenten Abschnitt (40) auszuwerten.

15. Anlage nach Anspruch 14, **dadurch gekennzeichnet, dass** die Messeinrichtung wenigstens eine Kamera (50) und ein Bildauswertungssystem (51) umfasst.

16. Anlage nach Anspruch 15, **dadurch gekennzeichnet, dass** die Messeinrichtung wenigstens eine fluoreszierende Folie (55) und wenigstens eine Quelle (53) für Anregungslicht umfasst.

17. Anlage nach einem der Ansprüche 11 bis 16, **dadurch gekennzeichnet, dass** die Anlage Bestandteil eines getaktet angetriebenen Fördersystems (60) ist, welches mehrere Förderelemente (61, 62), wobei jedes Förderelement (61, 62) zur Aufnahme mindestens eines Flüssigkeitskapillarfilters (1) vorgesehen ist, und wobei jedes Förderelement (61, 62) für jeden aufzunehmenden Flüssigkeitskapillarfilter (1) eine Blasenfalle (31) umfasst, welche gemeinsam mit dem Flüssigkeitskapillarfilter (1) entlang des Fördersystems (60) bewegbar ist.

18. Anlage nach Anspruch 15 oder 16, **dadurch gekennzeichnet, dass** die Messeinrichtung eine erste Kamera (50') umfasst, welche angrenzend an einen Förderweg des Fördersystems (60) stationär angeordnet ist.

19. Anlage nach Anspruch 18, **dadurch gekennzeichnet, dass** die Messeinrichtung eine zweite Kamera (50") umfasst, welche angrenzend an den Förderweg des Fördersystems (60) stationär angeordnet ist.

20. Anlage nach Anspruch 18 oder 19, **dadurch gekennzeichnet, dass** die erste und/oder die zweite Kamera (50', 50") angrenzend an eine Rastposition des Fördersystems (60) angeordnet sind.

21. Anlage nach einem der Ansprüche 17 bis 20, **dadurch gekennzeichnet, dass** das Fördersystem ein Rundförderer (60) ist.

## Claims

1. Method for checking the integrity of a hollow-fibre fluid filter (1), in particular a hollow-fibre dialyzer, which is constructed from a plurality of hollow fibres (15) enclosed by a membrane, with the steps of:
- perfusing the inside of the hollow fibres (15) with a fluid and
- supplying the outside of the hollow fibres (15) with a gas, or
- perfusing the outside of the hollow fibres (15) with a fluid and
- supplying the inside of the hollow fibres (15) with a gas,
wherein in each case the gas has a higher pressure than the fluid, and
- determining a quantity of the gas which penetrates into the fluid through holes in the membrane,
**characterized in that**,
- after flowing through the hollow-fibre fluid filter (1), the fluid is channelled through a bubble trap (30), and **in that**
- a gas volume (41) collecting in the bubble trap (30) during a predefined or predefinable reference period is determined.

2. Method according to claim 1, **characterized in that**
- an initial value of the gas volume (41) is determined at the start of the reference period,
- a final value of the gas volume (41) is determined at the end of the reference period, and
- **in that** the quantity of gas that has penetrated into the fluid in the reference period is determined from the difference between the initial value and the final value.

3. Method according to claim 1 or 2, **characterized in that** water is used as fluid.

4. Method according to one of claims 1 to 3, **characterized in that** a line system comprising the bubble trap (30) and the hollow-fibre fluid filter (1) is supplied with steam before the start of the check.

5. Method according to one of claims 1 to 4, **characterized in that** the determination of the volume of gas collected in the bubble trap (30) is effected visually.

6. Method according to claim 5, **characterized in that** the bubble trap (30) has a transparent section (40), and **in that**, for the determination of the volume of gas (41) collected in the bubble trap (30), the location of a fluid surface (42) in the transparent section (40) is assessed.

7. Method according to claim 6, **characterized in that**
- at least one first camera image of the transparent section (40) is captured at the start of the reference period and the initial location of the fluid surface (42) is determined by means of an image evaluation method,
- at least one second camera image of the transparent section (40) is captured at the end of the reference period and the final location of the fluid surface (42) is determined by means of an image evaluation method, and **in that**
- the volume of gas (41) collected is determined from the initial location of the fluid surface (42) and the final location of the fluid surface (42).

8. Method according to claim 7, **characterized in that** the first and the second camera image are captured using a transmitted light method.

9. Method according to claim 8, **characterized in that** the illumination is effected by means of a fluorescent film (55), which is illuminated with excitation light of a first wavelength, and which, in response to the illumination with excitation light, emits fluorescent light of a second wavelength, wherein the second wavelength is greater than the first wavelength, and wherein the camera images are captured through a filter (56) that is not transparent for light of the first wavelength.

10. Method according to one of claims 2 to 9, **characterized in that** a flow system comprising the bubble trap (30) and the hollow-fibre fluid filter (1) is arranged on a conveyor (60) operated in a pulsed manner, which is moved on at least one conveying position during the reference period.

11. Equipment for checking the integrity of a hollow-fibre fluid filter (1), in particular a hollow-fibre dialyzer, which is constructed from a plurality of hollow fibres (15) enclosed by a membrane, comprising:
- a flushing system which is set up to channel a fluid through the interior of the hollow fibres (15) of a hollow-fibre fluid filter (1) to be checked and
- a gas pressure system which is set up to supply the exterior of the hollow fibres (15) of the hollow-fibre fluid filter (1) to be checked with a gas, or
- a flushing system which is set up to channel a fluid through the exterior of the hollow fibres (15) of a hollow-fibre fluid filter (1) to be checked and
- a gas pressure system which is set up to supply the interior of the hollow fibres (15) of the hollow-fibre fluid filter (1) to be checked with a gas,
wherein in each case the pressure of the gas is higher than the pressure of the fluid, and
- a measuring device for the determination of a quantity of gas (41) penetrating into the fluid through holes in the membrane during a predefined or predefinable reference period,
**characterized in that**
- the flushing system comprises a bubble trap (30) arranged downstream of the hollow-fibre fluid filter (1), and
- **in that** the measuring device is set up to determine a volume of gas (41) collecting in the bubble trap (30) during a predefined or predefinable reference period.

12. Equipment according to claim 11, **characterized in that** the measuring device is set up
- to determine an initial value of the gas volume (41) at the start of the reference period,
- to determine a final value of the gas volume (41) at the end of the reference period, and
- to determine the quantity of gas that has penetrated into the hollow fibres (15) in the reference period from the difference between the initial value and the final value.

13. Equipment according to claim 11 or 12, **characterized in that** the measuring device is set up to determine the volume of gas (41) collecting in the bubble trap (30) visually.

14. Equipment according to claim 13, **characterized in that** the bubble trap (30) has a transparent section (40), and **in that**, for the determination of the volume of gas (41) collected in the bubble trap (30), the measuring device is set up to assess the location of a fluid surface (42) in the transparent section (40).

15. Equipment according to claim 14, **characterized in that** the measuring device comprises at least one camera (50) and an image evaluation system (51).

16. Equipment according to claim 15, **characterized in that** the measuring device comprises at least one fluorescent film (55) and at least one source (53) for excitation light.

17. Equipment according to one of claims 11 to 16, **characterized in that** the equipment is a component of a conveying system (60) driven in a pulsed manner, which [comprises] several conveying elements (61, 62), wherein each conveying element (61, 62) is provided for receiving at least one hollow-fibre fluid filter (1), and wherein each conveying element (61, 62) comprises, for each hollow-fibre fluid filter (1) to be received, a bubble trap (31) which is movable along the conveying system (60) together with the hollow-fibre fluid filter (1).

18. Equipment according to claim 15 or 16, **characterized in that** the measuring device comprises a first camera (50') which is arranged stationary adjacent to a conveying path of the conveying system (60).

19. Equipment according to claim 18, **characterized in that** the measuring device comprises a second camera (50") which is arranged stationary adjacent to the conveying path of the conveying system (60).

20. Equipment according to claim 18 or 19, **characterized in that** the first and/or the second camera (50', 50") are arranged adjacent to a stopping position of the conveying system (60).

21. Equipment according to one of claims 17 to 20, **characterized in that** the conveying system is a circular conveyor (60).

## Revendications

1. Procédé permettant de tester l'intégrité d'un filtre capillaire à liquide (1), en particulier d'un dialyseur capillaire, qui est constitué d'une pluralité de capillaires (15) entourés d'une membrane, comprenant les étapes suivantes :
- le passage d'un liquide à travers la face interne des capillaires (15) et
- l'exposition de la face externe des capillaires (15) à un gaz, ou
- le passage d'un liquide à travers la face externe des capillaires (15) et
- l'exposition de la face interne des capillaires (15) à un gaz, dans lequel le gaz est respectivement à une pression supérieure à celle du liquide, et
- la détermination d'une quantité de gaz qui pénètre dans le liquide à travers des fuites de la membrane, **caractérisé en ce que**
- le liquide est dirigé à travers un piège à bulles (30) après avoir traversé le filtre capillaire à liquide (1), et **en ce que**
- un volume de gaz (41) s'accumulant dans le piège à bulles (30) pendant une période de référence prédéterminée ou pouvant être prédéterminée est déterminé.

2. Procédé selon la revendication 1, **caractérisé en ce que**
- une valeur initiale du volume de gaz (41) est déterminée au début de la période de référence,
- une valeur finale du volume de gaz (41) est déterminée à la fin de la période de référence, et
- **en ce que**, à partir de la différence entre la valeur initiale et la valeur finale, on détermine la quantité de gaz qui a pénétré dans le liquide pendant la période de référence.

3. Procédé selon la revendication 1 ou 2, **caractérisé en ce que** l'on utilise de l'eau comme liquide.

4. Procédé selon l'une des revendications 1 à 3, **caractérisé en ce qu'**un système de conduites comprenant le piège à bulles (30) et le filtre capillaire à liquide (1) est exposé à de la vapeur d'eau avant le début de l'essai.

5. Procédé selon l'une des revendications 1 à 4, **caractérisé en ce que** la détermination du volume de gaz accumulé dans le piège à bulles (30) est réalisée par voie optique.

6. Procédé selon la revendication 5, **caractérisé en ce que** le piège à bulles (30) comporte une partie transparente (40), et **en ce que**, pour déterminer le volume de gaz (41) accumulé dans le piège à bulles (30), on évalue la position d'une surface de liquide (42) dans la partie transparente (40).

7. Procédé selon la revendication 6, **caractérisé en ce que**
- au début de la période de référence, au moins une première image de caméra de la section transparente (40) est prise et la position initiale de la surface de liquide (42) est déterminée au moyen d'un procédé d'évaluation d'image,
- à la fin de la période de référence, au moins une seconde image de caméra de la section transparente (40) est prise et la position finale de la surface de liquide (42) est déterminée au moyen d'un procédé d'évaluation d'image, et **en ce que**
- le volume de gaz accumulé (41) est déterminé à partir de la position initiale de la surface de liquide (42) et de la position finale de la surface de liquide (42).

8. Procédé selon la revendication 7, **caractérisé en ce que** la première et la seconde image de caméra sont prises par un procédé d'éclairage par transmission.

9. Procédé selon la revendication 8, **caractérisé en ce que** l'éclairage est réalisé au moyen d'une feuille fluorescente (55) qui est éclairée par une lumière d'excitation d'une première longueur d'onde et qui, en réponse à l'éclairage par la lumière d'excitation, émet une lumière fluorescente d'une seconde longueur d'onde, dans lequel la seconde longueur d'onde est supérieure à la première longueur d'onde, et dans lequel les images de la caméra sont prises à travers un filtre (56) opaque à la lumière de la première longueur d'onde.

10. Procédé selon l'une quelconque des revendications 2 à 9, **caractérisé en ce qu'**un système d'écoulement comprenant le piège à bulles (30) et le filtre capillaire à liquide (1) est disposé sur un convoyeur (60) fonctionnant de manière cadencée, lequel convoyeur est déplacé d'au moins une position de transport pendant la période de référence.

11. Installation permettant de tester l'intégrité d'un filtre capillaire à liquide (1), en particulier d'un dialyseur capillaire, qui est constitué d'une pluralité de capillaires (15) entourés d'une membrane, comprenant :
- un système de rinçage qui est configuré pour faire passer un liquide à travers l'intérieur des capillaires (15) d'un filtre capillaire à liquide (1) à tester, et
- un système de pression de gaz qui est configuré pour alimenter en gaz l'extérieur des capillaires (15) du filtre capillaire à liquide (1) à tester, ou
- un système de rinçage qui est configuré pour faire passer un liquide à travers l'extérieur des capillaires (15) d'un filtre capillaire à liquide (1) à tester, et
- un système de pression de gaz qui est configuré pour alimenter en gaz l'intérieur des capillaires (15) du filtre capillaire liquide (1) à tester, dans laquelle la pression du gaz est respectivement supérieure à la pression du liquide, et
- un dispositif de mesure pour déterminer une quantité de gaz (41) pénétrant dans le liquide pendant une période de référence prédéterminée ou pouvant être prédéterminée à travers des fuites de la membrane, **caractérisée en ce que**
- le système de rinçage comprend un piège à bulles (30) disposé en aval du filtre capillaire à liquide (1), et
- **en ce que** le dispositif de mesure est configuré pour déterminer un volume de gaz (41) s'accumulant dans le piège à bulles (30) pendant une période de référence prédéfinie ou pouvant être prédéfinie.

12. Installation selon la revendication 11, **caractérisée en ce que** le dispositif de mesure est configuré pour
- déterminer une valeur initiale du volume de gaz (41) au début de la période de référence,
- déterminer une valeur finale du volume de gaz (41) à la fin de la période de référence, et
- déterminer, à partir de la différence entre la valeur initiale et la valeur finale, la quantité de gaz qui a pénétré dans les capillaires (15) pendant la période de référence.

13. Installation selon la revendication 11 ou 12, **caractérisée en ce que** le dispositif de mesure est configuré pour déterminer optiquement le volume de gaz (41) qui s'accumule dans le piège à bulles (30).

14. Installation selon la revendication 13, **caractérisée en ce que** le piège à bulles (30) présente une section transparente (40), et **en ce que** le dispositif de mesure est configuré pour évaluer la position d'une surface de liquide (42) dans la section transparente (40) afin de déterminer le volume de gaz (41) accumulé dans le piège à bulles (30).

15. Installation selon la revendication 14, **caractérisée en ce que** le dispositif de mesure comprend au moins une caméra (50) et un système d'évaluation d'image (51).

16. Installation selon la revendication 15, **caractérisée en ce que** le dispositif de mesure comprend au moins une feuille fluorescente (55) et au moins une source (53) de lumière d'excitation.

17. Installation selon l'une des revendications 11 à 16, **caractérisée en ce que** l'installation fait partie d'un système de transport (60) entraîné de manière cadencée, qui comprend plusieurs éléments de transport (61, 62), dans laquelle chaque élément de transport (61, 62) est prévu pour recevoir au moins un filtre capillaire à liquide (1), et dans laquelle chaque élément de transport (61, 62) comprend, pour chaque filtre capillaire à liquide (1) à recevoir, un piège à bulles (31) qui peut se déplacer conjointement avec le filtre capillaire à liquide (1) le long du système de transport (60).

18. Installation selon la revendication 15 ou 16, **caractérisée en ce que** le dispositif de mesure comprend une première caméra (50') qui est disposée de manière stationnaire à proximité d'un trajet de transport du système de transport (60).

19. Installation selon la revendication 18, **caractérisée en ce que** le dispositif de mesure comprend une seconde caméra (50") qui est disposée de manière stationnaire et adjacente au trajet de transport du système de transport (60).

20. Installation selon la revendication 18 ou 19, **caractérisée en ce que** la première et/ou la seconde caméra (50', 50") sont disposées de manière adjacente à une position d'arrêt du système de transport (60).

21. Installation selon l'une quelconque des revendications 17 à 20, **caractérisée en ce que** le système de transport est un convoyeur circulaire (60).
